# EUROPÄISCHE PATENTSCHRIFT

(11) **EP 1 559 384 B1**
(45) Veröffentlichungstag und Bekanntmachung des Hinweises auf die Patenterteilung: **12.04.2006**
(21) Anmeldenummer: 04030219.2
(22) Anmeldetag: 21.12.2004
(51) Int. Cl.: A61F 2/60, A61F 2/68

(54) **Drehmomentsensor für eine Prothese**
Torque sensor for a prosthesis
Capteur de couple destiné a une prothèse

(30) Priorität: 29.01.2004 DE 102004004678
(43) Veröffentlichungstag der Anmeldung: 03.08.2005
(73) Patentinhaber: Otto Bock HealthCare GmbH, 37115 Duderstadt (DE)
(72) Erfinder: Boiten, Herman, Dipl.-Masch.-Ing., 37081 Göttingen (DE)
(74) Vertreter: Lins, Edgar

(56) Entgegenhaltungen:
- WO-A-00/17617
- DE-C1- 10 217 019
- US-A1- 2002 052 663
- US-A1- 2003 029 247
- SANDERS J E ET AL: "MEASUREMENT OF STRESSES IN THREE ORTHOGONAL DEIRECTIONS AT THE RESIDUAL LIMB-PROSTHETIC SOCKET INTERFACE" IEEE TRANSACTIONS ON REHABILITATION ENGINEERING, IEEE INC. NEW YORK, US, Bd. 1, Nr. 2, 1. Juni 1993 (1993-06-01), Seiten 79-85, XP000411672 ISSN: 1063-6528

## Beschreibung

Die Erfindung betrifft einen Drehmomentsensor für die Messung eines Drehmoments an einem vorgegebenen Ort eines Bauelements, insbesondere einer Prothese.

Die Erfindung befasst sich somit mit der Messung eines Drehmoments an einem bestimmten Ort eines Bauelements, um entweder lediglich die Information über das an diesem Ort auftretende Drehmoment für Warnzwecke zu erhalten oder mit der Information über das auftretende Drehmoment eine Steuerung vorzunehmen. Grundsätzlich ist es bekannt, Drehmomente mit geeigneten Messelementen, beispielsweise Dehnungsmessstreifen, zu ermitteln, die an den Stellen angebracht sind, wo das Drehmoment auftritt. In vielen Fällen bringt die Anordnung eines Drehmomentsensors an der Messstelle erhebliche konstruktive Nachteile mit sich, sodass dann nach weniger geeigneten konstruktiven Lösungen gesucht wird oder die Messung an einem anderen, weniger geeigneten Ort vorgenommen wird.

Ein Beispiel hierfür ist die Steuerung eines künstlichen Kniegelenks. Derartige künstliche Kniegelenke werden in der Standphase mit einer hohen Dämpfung versehen, um das Gelenk in der Standphase stabil zu halten. Demgegenüber muss bei einer beabsichtigten Beugung des Kniegelenks eine niedrige Dämpfung wirksam sein. Zur Steuerung der Dämpfung ist es bekannt, in der Nähe des Kniegelenks das Drehmoment um die Knieachse oder in der Nähe der Knieachse zu ermitteln. Solch ein Drehmomentsensor ist aus der Patentschrift EP 1285640 bekannt. Da sich jedoch sowohl beim Auftreten als auch kurz sich jedoch sowohl beim Auftreten als auch kurz vor dem Einbeugen des Kniegelenks ein etwa gleich großes Kniestreckmoment ausbildet, ist eine saubere Trennung zwischen Standphase und Schwungphase schwierig zu realisieren. Dadurch wird die Empfindlichkeit der Steuerung negativ beeinflusst.

Es ist bekannt, dass eine bessere Differenzierung zwischen der Standphase und der Schwungphase möglich wird, wenn ein Drehmoment direkt oberhalb des Fußes ermittelt wird, um ein dem Knöchelmoment ähnliches Signal zu liefern. Die Anordnung des Sensors im Unterschenkelbereich hat jedoch den Nachteil, dass der Aufbau des Unterschenkelrohres einer modularen Unterschenkelprothese in spezieller Weise erfolgen muss, sodass ein stark erhöhter konstruktiver Aufwand erforderlich wird. Zudem können Prothesenfüße mit integriertem Unterschenkelpylon nicht eingesetzt werden.

Der vorliegenden Erfindung liegt daher die Aufgabe zugrunde, einen Drehmomentsensor so auszubilden, dass konstruktive Nachteile durch seine Anordnung in dem Bauelement vermieden oder zumindest verringert werden.

Zur Lösung dieser Aufgabe ist erfindungsgemäß ein Drehmomentsensor der eingangs erwähnten Art dadurch gekennzeichnet, dass er als eine eine virtuelle Drehachse außerhalb des Sensors bildende Sensorstruktur aufgebaut ist und mit Verformungen der Sensorstruktur erfassenden Messaufnehmern zur Bestimmung eines Drehmoments um die virtuelle Drehachse versehen ist.

Der erfindungsgemäße Drehmomentsensor ermöglicht somit die Feststellung eines Drehmoments an einem außerhalb der Sensorstruktur liegenden Ort, der mit der virtuellen Drehachse der Sensorstruktur zusammenfällt. Hierzu weist der Drehmomentsensor für seine Verformung im Wesentlichen einen Freiheitsgrad auf.

Es ist - insbesondere auf dem Gebiet der Prothetik - bekannt, Mehrachs-Drehgelenke mit einer außerhalb des Gelenks angeordneten und sich während der Gelenkaktion bewegenden virtuellen Drehachse zu verwenden. Eine derartige Mehrachs-Struktur ist beispielsweise für Exartikulations-Amputierte erforderlich, weil sich in diesem Fall aus konstruktiven Gründen eine Knieachse nicht an der anatomisch richtigen Stelle unmittelbar unterhalb des Oberschenkelstumpfes anbringen lässt. Darüber hinaus kann die wandernde virtuelle Drehachse ausgenutzt werden, um die gewünschte Standstabilität zu erhöhen und die Beugung während der Schwungphase zu unterstützen.

Die erfindungsgemäße Sensorstruktur kann in analoger Weise aufgebaut sein, wobei jedoch nur eine sehr geringe Rotation der Sensorstruktur um die virtuelle Drehachse, also nur eine vernachlässigbare Verschiebung der Drehsachse bei Belastung, zugelassen wird. Dazu können beispielsweise die frei beweglichen Drehachsen der Mehrachs-Struktur durch nur geringfügig biegbare elastische Gelenke ersetzt werden.

Eine andere geeignete Struktur kann mit zwei starren, ggf. etwa parallel angeordneten Lenkern realisiert werden, die mittels zweier, einen Winkel miteinander bildenden Blattfedern verbunden sind. Die Schnittlinie der durch die Blattfedern aufgespannten Ebenen bildet dabei die virtuelle Drehachse außerhalb der Sensorstruktur.

In allen Ausführungsformen lässt sich das Drehmoment um die virtuelle Drehachse mittels einer entsprechenden Verformung der Sensorstruktur durch geeignete Messaufnehmer ermitteln. Geeignete Messaufnehmer können Kraft-, Druck- und/oder Wegaufnehmer sein. Besonders geeignet ist die Verwendung von Dehnungsmessstreifen.

Mit dem erfindungsgemäßen Drehmomentsensor lässt sich bevorzugt die Gelenkaktion einer Prothese steuern. Die Sensorstruktur kann insbesondere zur Steuerung eines künstlichen Kniegelenks im Kniebereich angeordnet werden und das Drehmoment im Unterschenkelbereich der Prothese messen.

Durch die Erfindung ist es ferner nicht ausgeschlossen, auch ein Drehmoment nicht nur außerhalb der Sensorstruktur, sondern auch außerhalb des Bauelements zu ermitteln, um hiermit ggf. verbesserte Steuerungen vorzunehmen.

Die Erfindung soll im Folgenden anhand von in der Zeichnung dargestellten Ausführungsbeispielen näher erläutert werden. Es zeigen:
- Figur 1 -: - eine modular aufgebaute Kniegelenkprothese mit einem künstlichen Fuß und einem Drehmomentsensor unmittelbar unterhalb des Kniegelenks, der eine virtuelle, das Unterschenkelrohr schneidende Drehachse der Prothese aufweist
- Figur 2 -: eine Anordnung gemäß Figur 1 mit einem Drehmomentsensor, dessen virtuelle Drehachse im Unterschenkelbereich außerhalb des Bauelements zwischen Vorfuß und Kniegelenksdrehachse angeordnet ist
- Figur 3 -: eine Seitenansicht eines in den Anordnungen gemäß Figur 1 bzw. Figur 2 verwendbaren Sensors
- Figur 4 -: eine perspektivische Ansicht des Sensors gemäß Figur 3
- Figur 5: - eine Seitenansicht des Sensors gemäß Figur 3 in einem verformten Zustand
- Figur 6 -: eine weitere Ausführungsform eines Drehmomentsensors, der mit Blattfedern aufgebaut ist
- Figur 7 -: eine perspektivische Ansicht des Drehmomentsensors gemäß Figur 6
- Figur 8 -: eine Seitenansicht des Drehmomentsensors in einem verformten Zustand.

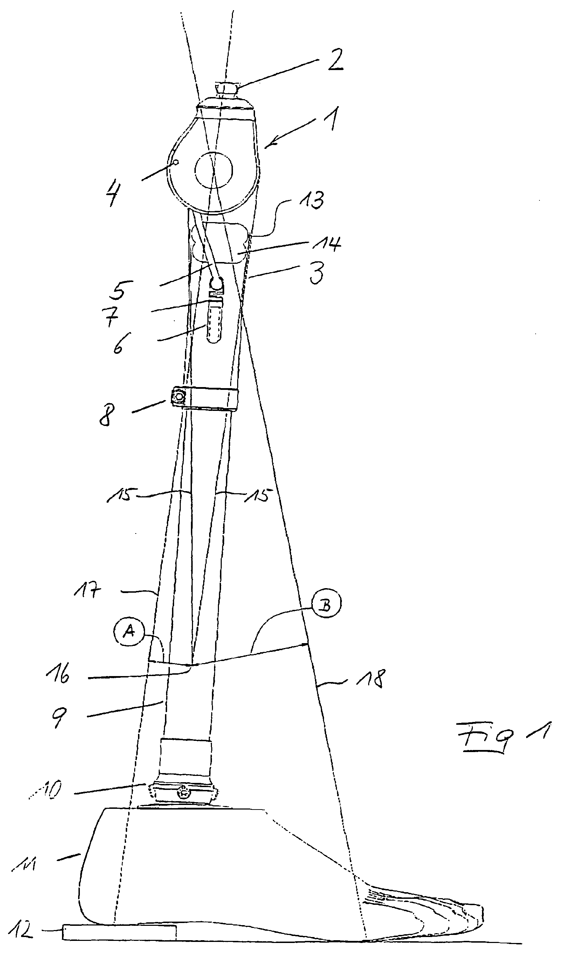

Die in Figur 1 dargestellte Prothese weist ein monoaxiales Kniegelenk 1 auf, das mit einem Anschlussadapter 2 mit einem (nicht dargestellten) Oberschenkel-Prothesenteil verbindbar ist. Das Kniegelenk 1 weist an seiner Unterseite einen Rohransatz 3 auf, in dem ein mit einer exzentrischen Drehachse 4 verbundener Lenker 5 in einer Längsführung 6 gegen die Rückstellkraft einer Druckfeder 7 verschiebbar gelagert ist. Der Lenker 5 wird bei einer Beugebewegung des Kniegelenks 1 gegen die Verstellkraft der Rückstellfeder 7 nach unten verschoben, bis der Anlenkpunkt der Hilfsachse 4 einen unteren Totpunkt überwindet. Daraufhin unterstützt die Rückstellkraft der Druckfeder 7 die weitere Beugung und stabilisiert das Knie in der gebeugten Stellung, wenn sich der Prothesenträger beispielsweise gesetzt hat.

Über eine Rohrmuffe 8 ist das Kniegelenk 1 mit einem Unterschenkelrohr 9 verbunden, dessen anderes Ende mit einer Justiermuffe 10 mit einem Justierzapfen eines künstlichen, gelenklosen Fußes 11 verbunden ist. Zur Verdeutlichung der Gebrauchsstellung des künstlichen Fußes 11 ist dieser im Fersenbereich auf einen kleinen Sockel 12 gestellt gezeichnet, der einem üblicherweise vorhandenen Schuhabsatz entspricht.

In den Rohransatz 3 des Kniegelenks 1 ist unterhalb der Drehachse eine Sensorstruktur 13 eingesetzt, deren Aufbau unten näher erläutert wird. Figur 1 lässt erkennen, dass die hohl aufgebaute Sensorstruktur aufgrund einer speziellen, inneren Kontur 14 und ihrer äußeren Kontur vier Stellen aufweist, an denen das Material verdünnt ist und die somit als elastische Gelenke fungieren. Vertikale Verbindungslinien 15 dieser Gelenke schneiden sich in einer virtuellen Drehachse 16, die in dem in Figur 1 dargestellten Ausführungsbeispiel mittig im Unterschenkelrohr 9 etwas oberhalb des Knöchelbereichs liegt.

In Figur 1 sind Abstände A, B der virtuellen Drehachse 16 zu Kraftwirkungslinien 17, 18 eingezeichnet, die den Auflagepunkt des Fußes 11 im Fersenbereich bzw. im Ballenbereich schneiden und durch die Drehachse des Kniegelenks 1 verlaufen. Die dargestellten Kraftwirkungslinien 17, 18 entsprechen etwa der Belastungssituation der Prothese beim Auftreten auf die Ferse 17 bzw. beim Abrollen auf dem Vorfuß 18.

Da der Abstand B zur Vorfuß-Kraftwirkungslinie 18 deutlich größer ist als der Abstand A zur Fersen-Kraftwirkungslinie 17, gehen auf den Vorfuß wirkende Kräfte mit einem größeren Hebelarm in die Drehmomentmessung des Sensors 13 ein als die auf die Ferse wirkenden Kräfte. Der in Figur 1 dargestellte Sensor 13 ist daher in erster Linie "vorfußempfindlich".

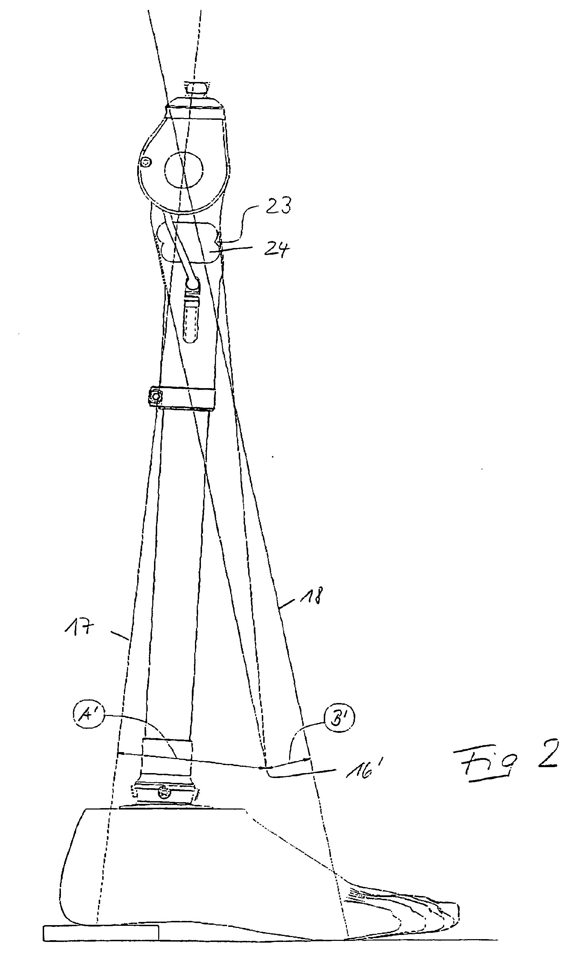

Die in Figur 2 dargestellte Anordnung entspricht der in Figur 1 dargestellten, jedoch mit dem Unterschied, dass der dargestellte Sensor 23 zwar an derselben Stelle eingebaut ist wie der Sensor 13 in Figur 1, jedoch aufgrund der Ausbildung seiner Innenkontur 24 und Außenkontur eine nach vorn verlagerte virtuelle Drehachse 16' aufweist. Somit ist der Abstand A' der virtuellen Drehachse 16' von der Fersen-Kraftwirkungslinie 17 wesentlich größer als der Abstand B' von der Vorfuß-Kraftwirkungslinie 18. Demzufolge ist der in Figur 2 dargestellte Sensor 23 "fersenempfindlich". Eine geeignete Positionierung der virtuellen Drehachse 16, 16' erleichtert die unter Benutzung der vom Sensor 13 erzeugten Messdaten durchgeführte Steuerung.

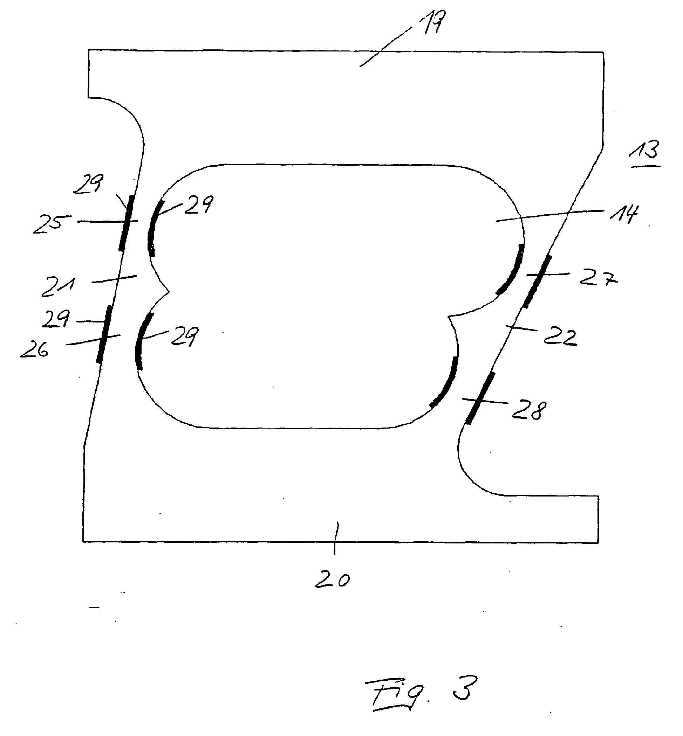

In den Figuren 3 bis 5 ist eine erste Ausführungsform einer Sensorstruktur 13 dargestellt. Diese besteht aus einem Block mit einem festen oberen Steg 19 und einem dazu parallel verlaufenden festen unteren Steg 20.

Der Innenraum 14 ist durch zwei vertikal verlaufende Stege 21, 22 begrenzt, die einen Winkel miteinander bilden, der dem Winkel der Verbindungslinien 15 in Figur 1 entspricht. Aufgrund der Gestaltung der Innenkontur 14 und Außenkontur weisen die Stege 21, 22 vier Stellen mit einer minimalen Materialdicke auf, die vier elastisch verformbare Gelenke 25, 26, 27, 28 bilden. Auf beiden Wandseiten der Gelenke 25, 26, 27, 28 sind jeweils innere und äußere Dehnmessstreifen 29 angebracht, mit denen Verformungen der elastisch verformbaren Gelenke 25, 26, 27, 28 messbar sind.

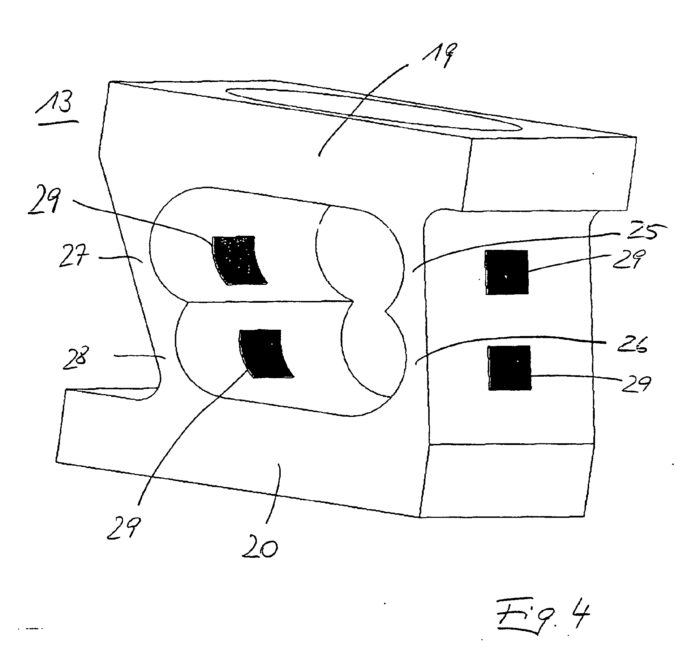

Figur 4 verdeutlicht die beschriebene Anordnung in einer perspektivischen Ansicht. Dabei wird deutlich, dass die Dehnmessstreifen 29 sich nicht über die gesamte Breite der Gelenke 27, 28, 29, 30 erstrecken, sondern eine wesentlich geringere Breite aufweisen und mittig bezüglich der Tiefe der Sensorstruktur 13 angeordnet sind.

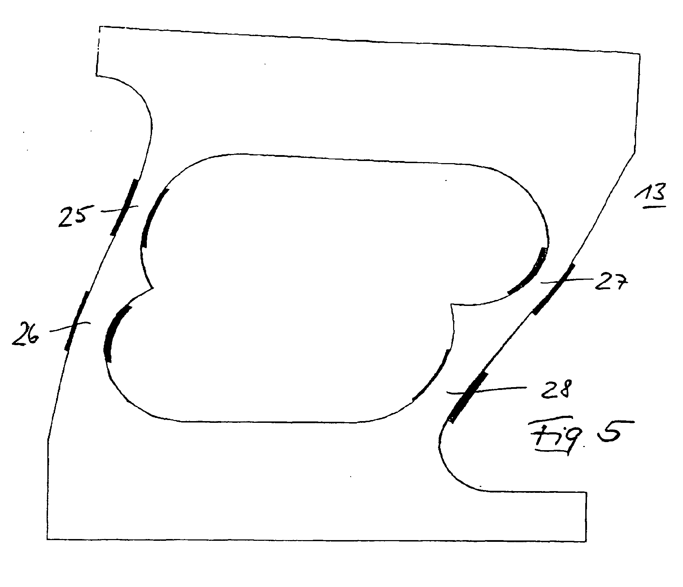

Figur 5 lässt im Vergleich zu Figur 3 eine leichte Drehung des oberen Stegs 19 relativ zum unteren Steg 20 erkennen. Eine vom Drehmoment um die virtuelle Drehachse 16 verursachte Drehung des oberen Stegs 19 bedingt eine übereinstimmende Drehung der elastischen Gelenke 25, 26, 27, 28. Die durch die Dehnungsmessstreifen 29 gemessene Verformung entspricht somit direkt einem Drehmoment, das um die virtuelle Drehachse 16 der Sensorstruktur 13 auftritt.

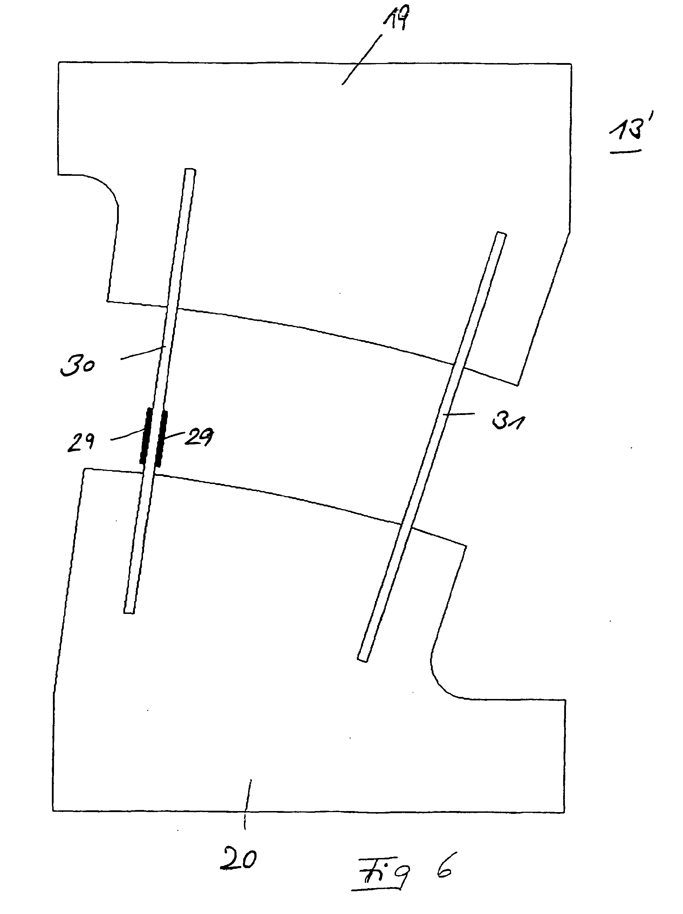

Die Figuren 6 bis 8 zeigen eine weitere Ausführungsform einer Sensorstruktur 13', bei der die Verbindung zwischen dem oberen Steg 19 und dem unteren Steg 20 mittels zweier eingespannter ebener Blattfedern 30, 31 erfolgt. Die Ebenen der Blattfedern 30, 31 entsprechen den Verbindungslinien 15 und bilden einen Winkel miteinander, dessen Schnittlinie die virtuelle Drehachse 16 bestimmt. In dem dargestellten Ausführungsbeispiel ist eine Blattfeder 30 mit einem inneren und einem äußeren Dehnungsmessstreifen 29 versehen.

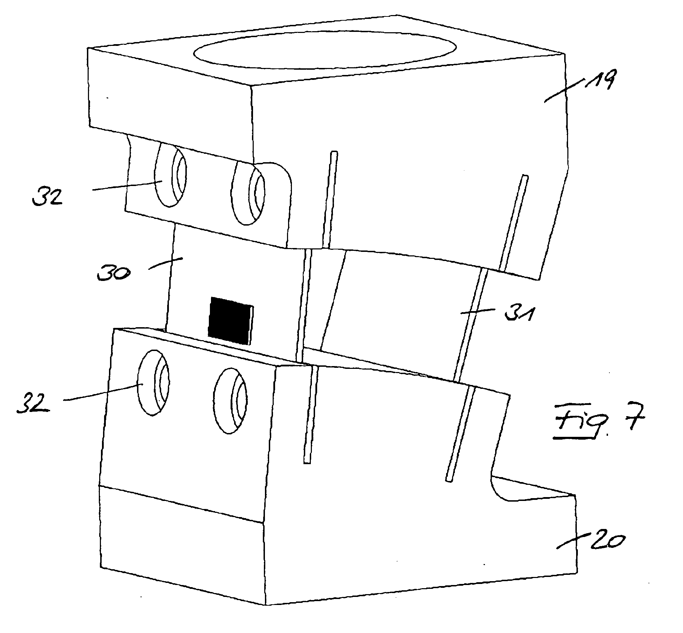

Die perspektivische Ansicht gemäß Figur 7 verdeutlicht Bohrungen 32 in den starren Stegen 19, 20, die der Befestigung bzw. Einspannung der Blattfedern 30, 31 dienen.

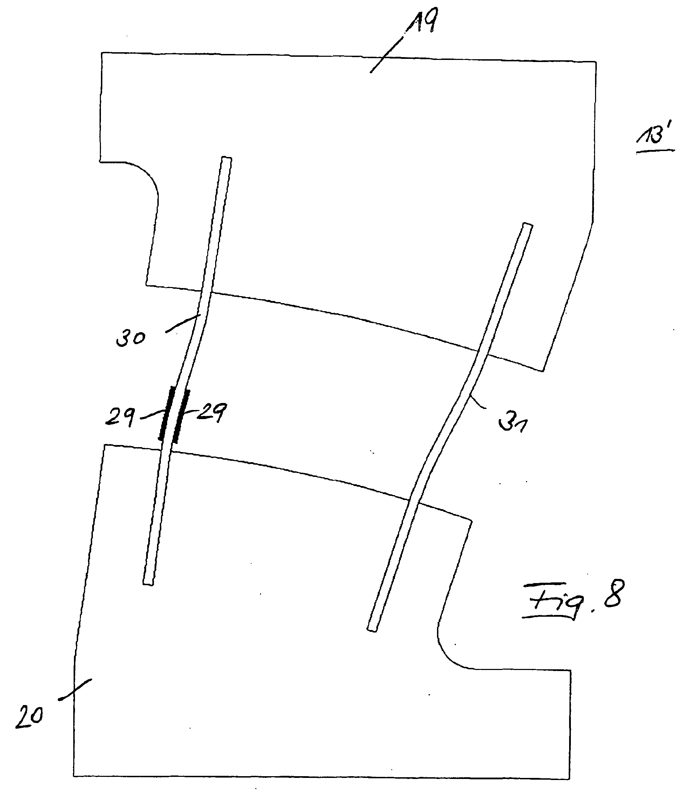

Figur 8 zeigt einen belasteten Zustand der Sensorstruktur 13', in dem die Blattfedern 30, 31 aufgrund eines Drehmoments in der virtuellen Drehachse 16 durch jeweils zwei Biegungen mit jeweils zwei Biegepunkten verformt sind, wodurch eine geringfügige Drehung des oberen starren Steges 19 relativ zum unteren starren Steg 20 erfolgt. Die Dehnungsmessstreifen 29 detektieren die Biegung der Blattfeder 30 an einem der Biegepunkte der Blattfeder 30. Diese Biegung steht in einem direkten Zusammenhang mit dem Drehmoment um die virtuelle Drehachse 16, 16' der Sensorstruktur 13. Auch die Biegungen an den anderen Biegestellen können zur Bestimmung des Drehmoments um die virtuelle Drehachse genutzt werden. Eine kombinierte Messung an mehreren Biegestellen kann die Genauigkeit der Messung ggf. erhöhen.

Während die Ausführungsform der Sensorstruktur 13 gemäß den Figuren 3 bis 5 als klare Vier-Gelenk-Struktur erkennbar ist, zeigt die Ausführungsform gemäß den Figuren 6 bis 8, dass auch andere Strukturen geeignet sind, für die erfindungsgemäße Drehmomentmessung außerhalb der Sensorstruktur 13, 13', 23 eingesetzt zu werden.

## Patentansprüche

1. Drehmomentsensor für die Messung eines Drehmoments an einem vorgegebenen Ort eines Bauelements, insbesondere einer Prothese, **dadurch gekennzeichnet, dass** er als eine eine virtuelle Drehachse (16, 16') außerhalb des Sensors bildende Sensorstruktur (13, 13', 23) aufgebaut ist und mit Verformungen der Sensorstruktur (13, 13', 23) erfassenden Messaufnehmern (29) zur Bestimmung eines Drehmoments um die virtuelle Drehachse (16, 16') versehen ist.

2. Drehmomentsensor nach Anspruch 1, **gekennzeichnet durch** eine Ausbildung als Mehrgelenkstruktur mit elastisch verformbaren Gelenken (25, 26, 27, 28).

3. Drehmomentsensor nach Anspruch 2, **gekennzeichnet durch** eine Ausbildung als Viergelenkstruktur.

4. Drehmomentsensor nach Anspruch 1, **gekennzeichnet durch** eine Ausbildung mit zwei planaren Blattfederanordnungen (30, 31), deren Ebenen sich in der virtuellen Drehachse (16, 16') schneiden.

5. Drehmomentsensor nach Ansprüche 1 bis 4, **dadurch gekennzeichnet, dass** die Messaufnehmer (29) Dehnungsmessstreifen sind.

6. Prothese mit einem zur Steuerung eines künstlichen Gelenks eingesetzten Drehmomentsensor nach einem der Ansprüche 1 bis 5.

7. Prothese nach Anspruch 6 mit einem Drehmomentsensor zur Steuerung eines künstlichen Kniegelenks (1), **dadurch gekennzeichnet, dass** die virtuelle Drehachse (16, 16') der Sensorstruktur (13, 13', 23) im Unterschenkelbereich der Prothese liegt und dass die Sensorstruktur (13, 13', 23) im Kniebereich angeordnet ist.

8. Prothese nach Anspruch 7 mit einem Drehmomentsensor zur Steuerung eines künstlichen Kniegelenks (1), **dadurch gekennzeichnet, dass** die virtuelle Drehachse (16, 16') der Sensorstruktur (13, 13', 23) im Knöchelbereich der Prothese liegt und dass die Sensorstruktur (13, 13', 23) im Kniebereich angeordnet ist.

## Claims

1. A torque sensor for measuring a torque at a predetermined location of a structural element, in particular of a prosthesis, **characterized in that**, it is constructed as a sensor structure (13, 13', 23) forming a virtual pivot axis (16, 16') outside the sensor and is provided with measurement transducers (29) which detect deformations of the sensor structure (13, 13', 23) for determining a torque about the virtual pivot axis (16, 16').

2. The torque sensor as claimed in claim 1, which is designed as a multi-hinge structure with elastically deformable hinges (25, 26, 27, 28).

3. The torque sensor as claimed in claim 2, which is designed as a four-hinge structure.

4. The torque sensor as claimed in claim 1, which is designed with two planar leaf-spring arrangements (30, 31) whose planes intersect in the virtual pivot axis (16, 16').

5. The torque sensor as claimed in claims 1 through 4, wherein the measurement transducers (29) are strain gauges.

6. A prosthesis with a torque sensor as claimed in one of claims 1 through 5 used for controlling an artificial joint.

7. The prosthesis as claimed in claim 6 with a torque sensor for controlling an artificial knee joint (1), wherein the virtual pivot axis (16, 16') of the sensor structure (13, 13', 23) lies in the below-knee area of the prosthesis, and the sensor structure (13, 13', 23) is arranged in the knee area.

8. The prosthesis as claimed in claim 7 with a torque sensor for controlling an artificial knee joint (1), wherein the virtual pivot axis (16, 16') of the sensor structure (13, 13', 23) lies in the ankle area of the prosthesis, and the sensor structure (13, 13', 23) is arranged in the knee area.

## Revendications

1. Capteur de couple pour mesurer un couple dans un endroit donné d'un composant, en particulier une prothèse, **caractérisé en ce qu'**il est réalisé avec un axe de rotation virtuel (16, 16') extérieur à la structure de capteur (13, 13', 23) constituant le capteur et comporte un capteur de mesure (29) détectant la déformation de la structure du capteur (13, 13', 23) pour déterminer un couple autour de l'axe de rotation virtuel (16, 16').

2. Capteur de couple selon la revendication 1, **caractérisé par** une réalisation sous forme de structure à plusieurs articulations élastiquement déformables (25, 26, 27, 28).

3. Capteur de couple selon la revendication 2, **caractérisé par** une réalisation sous forme de structure à quatre articulations.

4. Capteur de couple selon la revendication 1, **caractérisé par** une réalisation comportant deux agencements de lames de ressort (30, 31), dont les plans se coupent sur l'axe de rotation virtuel (16, 16').

5. Capteur de couple selon les revendications 1 à 4, **caractérisé en ce que** le récepteur de mesure (29) est constitué par des bandes de mesure d'allongement.

6. Prothèse comportant pour commander une articulation artificielle, un capteur de rotation selon l'une des revendications 1 à 5.

7. Prothèse selon la revendication 6 comportant un capteur de couple pour commander une articulation artificielle de genou (1), **caractérisée en ce que** l'axe de rotation virtuel (16, 16') de la structure de capteur (13, 13', 23) est situé dans la région de la jambe de la prothèse et la structure de capteur (13, 13', 23) est située dans la région du genou.

8. Prothèse selon la revendication 7 comportant un capteur de couple pour commander une articulation artificielle de genou (1), **caractérisée en ce que** l'axe de rotation virtuel (16, 16') de la structure de capteur (13, 13', 23) est situé dans la région de la cheville de la prothèse et la structure (13, 13', 23) est située dans la région du genou.
